# EUROPEAN PATENT APPLICATION

(11) **EP 3 034 047 A1**
(43) Date of publication of application: **22.06.2016**
(21) Application number: 13894382.4
(22) Date of filing: 27.09.2013
(51) Int. Cl.: A61F 2/915, A61F 2/88, A61L 31/00

(54) **STENT**

(71) Applicant: Terumo Kabushiki Kaisha, Tokyo 151-0072 (JP)
(72) Inventor: MITSUDO, Kazuaki, Kurashiki-shi Okayama 710-0048 (JP); KITO, Takayuki, Ashigarakami-gun Kanagawa 259-0151 (JP)
(74) Representative: Prüfer & Partner mbB Patentanwälte · Rechtsanwälte
(86) International application number: PCT/JP2013/076279
(87) International publication number: WO 2015/045101

(57) **Abstract**

There is provided a stent which can prevent linear configuration elements configuring the stent from being unexpectedly disconnected from each other, while achieving an expansion holding force and excellent flexibility of the stent.

A stent (10) has a stent body (20) that is configured to include linear configuration elements, and that has a cylindrical shape having a gap as a whole, a first connection portion (31) and a second connection portion (32) which are integrally formed with the stent body (20) in each of the linear configuration elements adjacent to each other side by side in an axial direction (X) of the stent body (20) having the cylindrical shape, and which are connected to each other while positions overlap in the axial direction (X) and in a circumferential direction (Y) of the stent, and a connection member (40) that includes a biodegradable material for connecting the first connection portion (31) and the second connection portion (32) to each other.

## Description

### [Technical Field]

The present invention relates to a stent which is caused to indwell a stenosis, an occluded portion, or the like appearing in a body lumen so as to maintain a patency state of the body lumen.

### [Background Art]

In recent years, for example, in order to treat myocardinal infarction or angina pectoris, a method has been used in which a stent is caused to indwell a lesion (stenosis) of a coronary artery so as to secure a space inside an artery. In some cases, in order to treat a stenosis appearing in other body lumens such as other blood vessels, biliary ducts, bronchial tubes, esophagi, and urethrae, a similar method has been used. The stent is classified into a balloon-expandable stent and a self-expandable stent, depending on functions and methods for stent indwelling.

In a case of the balloon-expandable stent, the stent itself has no expandable function. After the stent is mounted on an outer side of a balloon of a balloon catheter including a dilatable balloon and is inserted into a target portion, the stent is expanded and plastically deformed by the balloon. In this manner, the stent is fixedly and closely attached to the inside of the body lumen such as the blood vessel and the like. In contrast, in a case of the self-expandable stent, the stent itself has the expandable function. The stent is accommodated into a catheter in a state where the stent has a contracted diameter in advance. After reaching a target area, the stent is expanded by being pushed from the catheter so as to release the state having the contracted diameter. In this manner, the stent is fixedly and closely attached to the inside of the body lumen such as the blood vessel and the like.

The stent needs to be provided with a sufficient vasodilation holding force for holding a expanded state of the blood vessel. However, the vasodilation holding force (strength) held by the stent becomes a load, thereby causing a possibility of restenosis in the blood vessel. Accordingly, it is preferable that the stent becomes flexible after the stent indwells the blood vessel and a predetermined period of time elapses. Therefore, for example, PTL 1 discloses a stent in which portions adjacent to a metal wire-shaped body which is formed in a helical shape while being folded into a waved shape are connected to a biodegradable linear body. The stent configured in this way is provided with sufficient vasodilation holding force, and can satisfactorily improve stenosis. After the predetermined period of time elapses, the biodegradable linear body becomes biodegraded and flexible, thereby achieving improved following property along the deformation of the blood vessel.

In addition, PTL 2 discloses a stent in which apexes adjacent to a helical body which is formed in a helical shape while being folded into a waved shape are caused to face each other, and in which at least one connection portion for connecting the facing apexes by means of welding, soldering, or the like is provided while a helix is wound one turn. The stent configured in this way has the helical shape, and thus, the flexible helical body is provided with strength to some degree by the connection portion. Accordingly, the stent achieves following property along the deformation of the blood vessel while being provided with a sufficient vasodilation holding force.

### [Citation List]

### [Patent Literature]

[PTL 1] JP-A-2009-82245
[PTL 2] U.S. Patent No. 8366765

### [Summary of Invention]

### [Technical Problem]

However, according to the stent disclosed in PTL 1, for example, when the stent is crimped on the balloon or the like, when the stent passes through a lesion after being inserted into the blood vessel, or when the stent is operated so as to expand, the biodegradable linear body is disconnected from the metal wire-shaped body, thereby weakening vasodilation holding force of the stent. Furthermore, there is a possibility that the stent may be inhibited from expanding into a desired shape. In addition, according to the stent disclosed in PTL 2, the facing apexes are connected to each other by means of welding, soldering, or the like. Consequently, for example, when the stent is crimped on the balloon or the like, when the stent passes through a lesion after being inserted into the blood vessel, or when the stent is operated so as to expand, the stent is less likely to be disconnected. However, there is a possibility that the stent may have insufficient following property to move along the deformation of the blood vessel.

The present invention is made in order to solve the above-described problems, and an object thereof is to provide a stent which can prevent linear configuration elements configuring the stent from being unexpectedly disconnected from each other, while achieving expansion holding force and excellent flexibility of the stent.

### [Technical Problem]

In order to achieve the above-described object, a stent according to the present invention includes a stent body that is configured to include linear configuration elements, and that has a cylindrical shape having a gap as a whole, a first connection portion and a second connection portion which are integrally formed with the stent body in each of the linear configuration elements adjacent to each other side by side in an axial direction of the stent body having the cylindrical shape, and which are connected to each other while positions overlap in the axial direction and in a circumferential direction of the stent body, and a connection member that includes a biodegradable material for connecting the first connection portion and the second connection portion to each other.

### [Advantageous Effects of Invention]

A stent configured as described above includes a strengthened expansion holding force since linear configuration elements configuring the stent are connected to each other by a connection member, and the connection member includes a biodegradable material. Accordingly, the connection member is biodegraded after the stent indwells a body lumen and a predetermined period of time elapses, and a first connection portion and a second connection portion are disconnected from each other, thereby achieving excellent flexibility. Then, positions in an axial direction and a circumferential direction of the first connection portion and the second connection portion which are connected to each other overlap. Accordingly, for example, when the stent is crimped on a balloon, when the stent passes through a lesion after being inserted into a body lumen, or when the stent is operated so as to expand, it is possible to prevent the first connection portion and the second connection portion from being unexpectedly disconnected from each other.

If the connection member is a filling member for filling a portion between the first connection portion and the second connection portion which are connected to each other, the connection member can be firmly connected to the first connection portion and the second connection portion.

If at least one of the first connection portion and the second connection portion which are connected to each other has a protruding portion formed so as to protrude from the stent body, and if at least the other one of the first connection portion and the second connection portion has a recessed housing portion for housing the protruding portion, the housing portion houses the protruding portion. In this manner, the first connection portion and the second connection portion can be firmly connected to each other, and can be less likely to be disconnected from each other.

If the stent body includes multiple helical members formed in a helical shape wound around the stent body in the axial direction, and if the first connection portion and the second connection portion connect the adjacent helical members to each other, the stent is provided with flexibility by the helical member having a helical shape, and the stent is provided with proper rigidity by the connection portions. In this manner, the stent achieves improved following property along the deformation of the blood vessel while including a sufficient vasodilation holding force.

If the first connection portion and the second connection portion which are connected to each other are arranged so as to become close to each other when being twisted in a direction where the helix is tightened, when tensile force is applied to the first connection portion and the second connection portion in the direction where the helix is tightened, the force is received in the direction where the first connection portion and the second connection portion become close to each other. Accordingly, the first connection portion and the second connection portion are less likely to be disconnected from each other.

If the first connection portion and the second connection portion which are connected to each other are arranged so as to become close to each other when being twisted in a direction where the helix is loosened, when a tensile force is applied to the first connection portion and the second connection portion in the direction where the helix is loosened, the force is received in the direction where the first connection portion and the second connection portion become close to each other. Accordingly, the first connection portion and the second connection portion are less likely to be disconnected from each other.

If the helical members adjacent to each other in the axial direction are connected to each other only by the first connection portion and the second connection portion, except for a portion to be connected in the circumferential direction so as to configure the same helix, the helical members are not connected to each other by an integral structure extending in the axial direction, but are connected to each other in the axial direction only by the first connection portion and the second connection portion between which the filling member is interposed. Therefore, it is possible to improve following property along the deformation of a body lumen by improving flexibility of the stent.

If the stent body further includes an annular body formed endlessly in both ends of the helical member, it is possible to strengthen vasodilation holding force in both end portions of the stent.

If the first connection portion and the second connection portion have a through-hole to be filled with the connection member, the connection member can be more firmly connected to the first connection portion and the second connection portion, and the first connection portion and the second connection portion can be less likely to be disconnected from each other.

If the filling member includes a drug, the connection member is biodegraded, and the drug is gradually eluted. Accordingly, it is possible to prevent restenosis of the blood vessel.

If the stent body is formed of a non-biodegradable metal material, the stent can include more sufficient vasodilation holding force.

### [Brief Description of Drawings]

[Fig. 1] Fig. 1 is a plan view when a stent according to an embodiment contracts.
[Fig. 2] Fig. 2 is a deployment view when the stent according to the embodiment contracts.
[Fig. 3] Fig. 3 is an enlarged plan view illustrating a part when the stent according to the embodiment contracts.
[Fig. 4] Fig. 4 is a sectional view taken along line 4-4 in Fig. 3.
[Fig. 5] Fig. 5 is a deployment view when the stent according to the embodiment expands.
[Fig. 6] Fig. 6 is an enlarged plan view illustrating a portion when the stent according to the embodiment expands.
[Fig. 7] Fig. 7 is an enlarged plan view illustrating a part when the stent according to the embodiment contracts.
[Fig. 8] Fig. 8 is a sectional view illustrating a modification example of the stent according to the embodiment.
[Fig. 9] Fig. 9 is a sectional view illustrating a modification example of the stent according to the embodiment.
[Fig. 10] Fig. 10 is a sectional view illustrating a modification example of the stent according to the embodiment.
[Fig. 11] Fig. 11 is a plan view illustrating a balloon catheter for causing the stent to indwell a body lumen.
[Fig. 12] Fig. 12 is a sectional view illustrating a distal portion of the balloon catheter.
[Fig. 13] Fig. 13 is a sectional view illustrating the distal portion of the balloon catheter when a balloon dilates.
[Fig. 14] Fig. 14 is a plan view illustrating a modification example of the stent according to the embodiment.

### [Description of Embodiments]

Hereinafter, an embodiment according to the present invention will be described with reference to the drawings. In some cases, a dimension ratio in the drawings may be exaggerated and different from a ratio used in practice in order to facilitate the description.

A stent 10 according to the present embodiment is used in order to treat stenosis or an occluded portion appearing in blood vessels, biliary ducts, bronchial tubes, esophagi, urethrae, or other body lumens. In this description, a side inserted into a lumen is referred to as a "distal end" or a "distal side", and an operating hand side is referred to as a "proximal end" or a "proximal side".

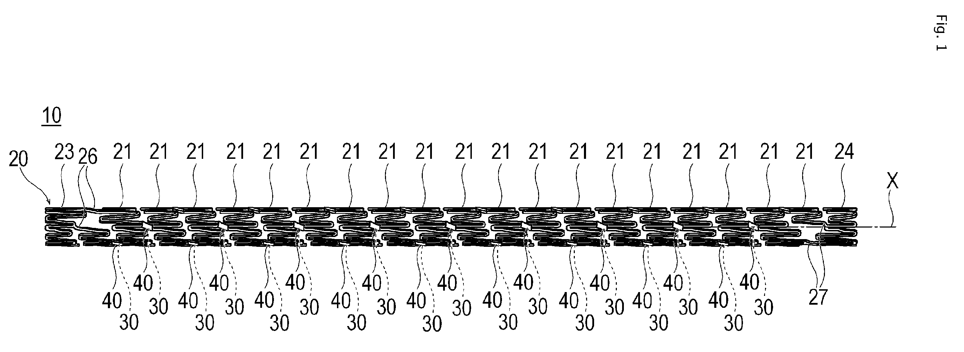

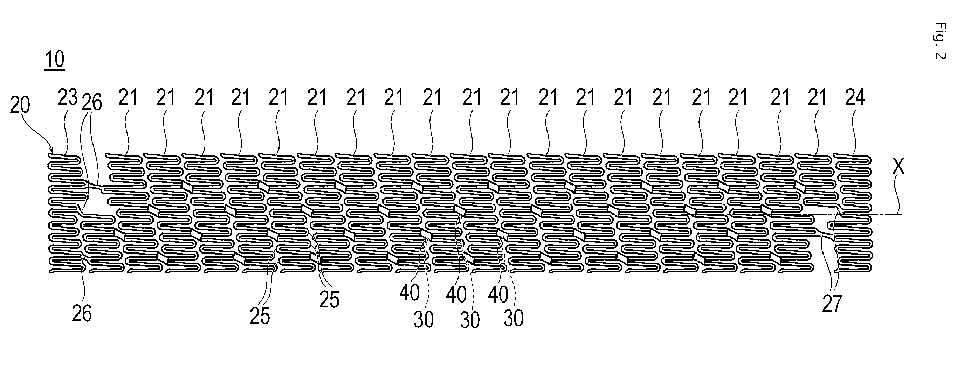

The stent 10 is a so-called balloon-expandable stent which expands using balloon-dilating force. As illustrated in Figs. 1 to 4, the stent 10 includes a stent body 20 that is configured to include linear configuration elements, and that has a cylindrical shape having a gap as a whole, connection portions 30 that connect the linear configuration elements adjacent to each other side by side in an axial direction X of the stent body 20 having the cylindrical shape, and a connection member 40 that connects the connection portions 30 to each other. In the stent 10 according to the present embodiment, the connection member 40 is formed of a filling member for filling a portion between the connection portions 30 which are connected to each other.

The stent body 20 includes multiple helical members 21 which are formed in a helical shape wound around the stent 10 in the axial direction X by the linear configuration elements having a zigzag shape folded into a waved shape, and annular bodies 23 and 24 which are formed endlessly in both ends of the helical member 21.

The respective helical members 21 are formed so as to draw a helix of 360°, and all of the helical members are arranged side by side in series, thereby configuring one helix. The respective helix members 21 include a folded portion 25 which protrudes by being folded into a waved shape. The number of the helical members 21 is not particularly limited. In addition, the number of the folded portions 25 disposed in the respective helical members 21 is also not particularly limited. Since the stent body 20 includes the helical member 21 formed in a helical shape, the stent 10 is provided with flexibility. Accordingly, the stent 10 is likely to be deformed so as to follow along the deformation of a body lumen such as the blood vessel and the like. Therefore, it is possible to reduce the influence on the living body.

The helical member 21 disposed on one end side of the stent 10 and an annular body 23 adjacent to the helical member 21 are integrally connected to each other by a ring member 26 which is a linear configuration element. In addition, the helical member 21 disposed on the other end side of the stent 10 and an annular body 24 adjacent to the helical member 21 are integrally connected to each other by a ring member 27 which is a linear configuration element. Since both end portions of the stent body 20 include the annular bodies 23 and 24 formed endlessly, even after the connection member 40 is biodegraded, a vasodilation holding force is strongly maintained in both end portions of the stent 10.

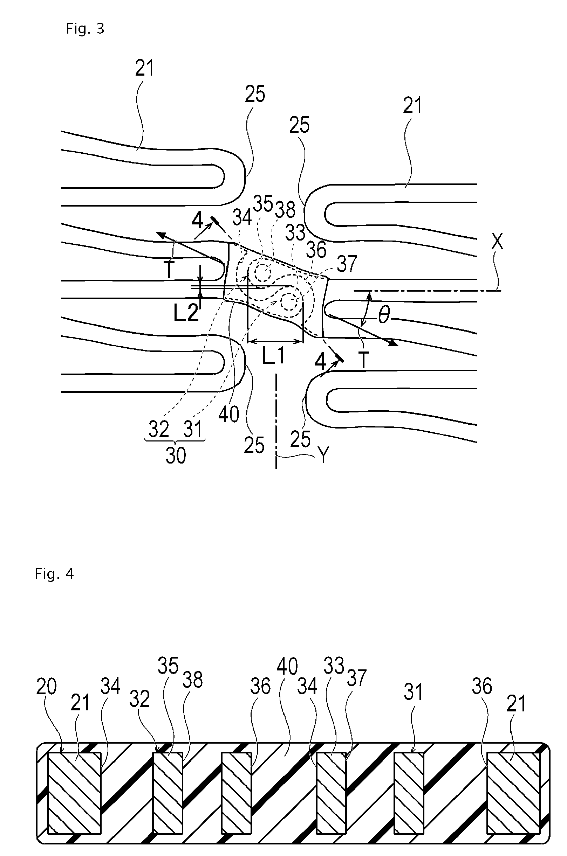

As illustrated in Figs. 3 and 4, the connection portion 30 includes a first connection portion 31 which is integrally formed on one side of the helical member 21 adjacent side by side in the axial direction X of the stent body 20, and a second connection portion 32 which is integrally formed on the other side of the adjacent helical member 21. The first connection portion 31 and the second connection portion 32 are respectively formed in the folded portions 25 which face each other in the adjacent helical members 21. It is preferable to dispose one or more connection portions 30 in the respective helical members 21 (within a range of 360° in the helix). The helical members 21 adjacent to each other in the axial direction X are connected to and integrally formed with each other in a circumferential direction Y so as to configure the same helix, and are connected to each other in the axial direction X only by the connection portions 30 between which the connection member 40 is interposed, except for a portion connected in the circumferential direction Y. That is, the helical members 21 adjacent to each other in the axial direction X are not connected to each other so as to be continuously linked in the axial direction X by an integral configuration with the stent body 20 such as the ring members 26 and 27, but are connected to each other in the axial direction X only by the connection portions 30 between which the connection member 40 is interposed. The helical members 21 adjacent to each other in the axial direction X can be partially connected to each other so as to be continuously linked in the axial direction X by the integral configuration with the stent body 20 such as the ring members 26 and 27.

The first connection portion 31 includes a first protruding portion 33 which is formed to protrude from a zigzag shape formed in a waved shape of the stent body 20, and a first housing portion 34 which is formed in a recessed shape. The second connection portion 32 includes a second protruding portion 35 which is formed to protrude from a zigzag shape formed in a waved shape of the stent body 20 and which is housed in the first housing portion 34, and a second housing portion 36 which has a recessed shape for housing the first protruding portion 33. The first protruding portion 33 is housed in the second housing portion 36 so as to have a gap, and the second protruding portion 35 is housed in the first housing portion 34 so as to have a gap. The first protruding portion 33 may come into partial contact with the second housing portion 36, and the second protruding portion 35 may come into partial contact with the first housing portion 34.

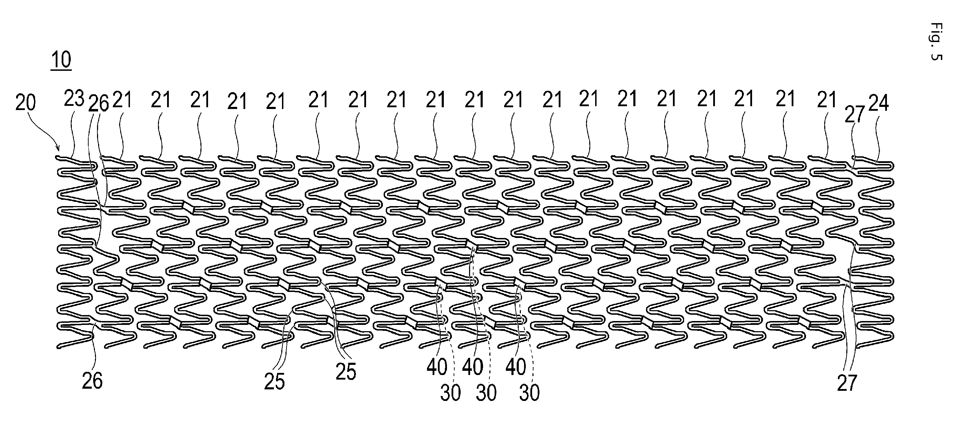

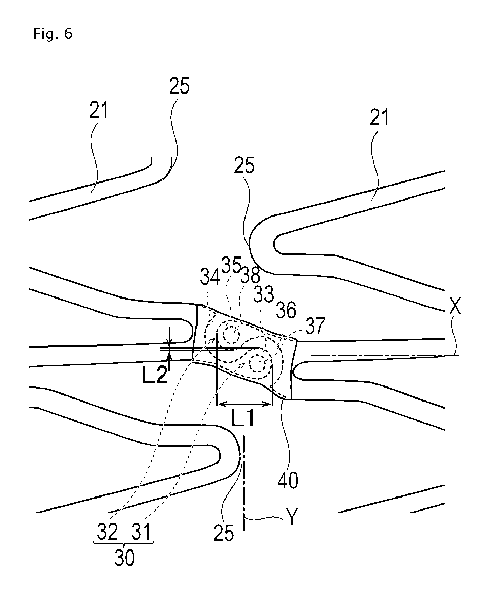

In a state where the stent 10 contracts, positions of the first connection portion 31 and the second connection portion 32 in the axial direction X of the stent 10 overlap by an amount of an axially overlapped length L1. The axially overlapped length L1 may be changed when the stent 10 expands. However, it is preferable that the axially overlapped length L1 is present (positions overlap) in all states from a contracted state to an expanded state (refer to Figs. 5 and 6).

Furthermore, in a state where the stent 10 contracts, positions of the first connection portion 31 and the second connection portion 32 in the circumferential direction Y of the stent 10 overlap by an amount of a circumferentially overlapped length L2. The circumferentially overlapped length L2 may be changed when the stent 10 expands. However, it is preferable that the circumferentially overlapped length L2 is present (positions overlap) in all states from a contracted state to an expanded state (refer to Figs. 5 and 6).

In this way, since the first connection portion 31 and the second connection portion 32 overlap at the positions in the axial direction X and the circumferential direction Y of the stent 10, when the stent 10 expands, the connection between the first connection portion 31 and the second connection portion 32 is satisfactorily maintained so as to be caught on each other. Accordingly, it is possible to prevent the first connection portion 31 and the second connection portion 32 from being disconnected from each other.

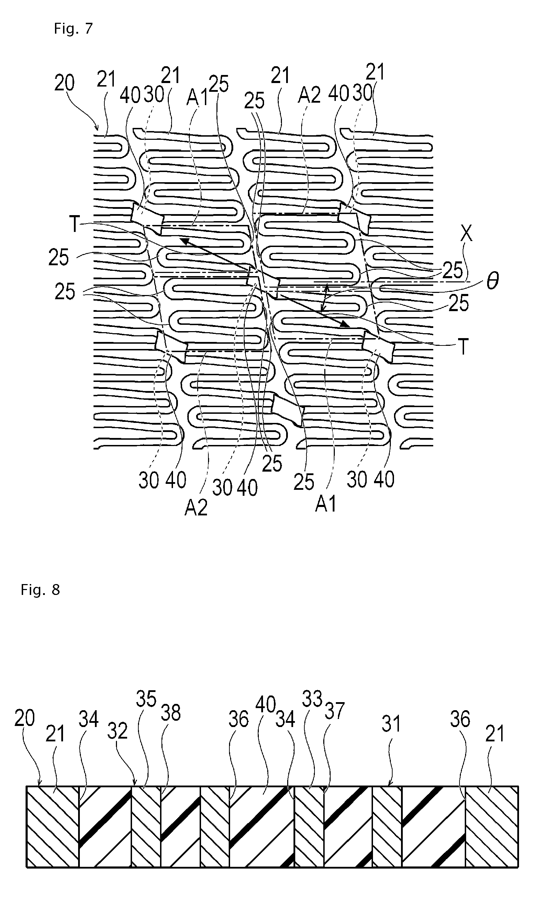

In the stent 10 according to the present embodiment, when the stent 10 expands, tensile force is applied to the first connection portion 31 and the second connection portion 32 in a tensile direction T which tilts at an angle θ with respect to the axial direction X. For example, as in a range illustrated by a chain line in Fig. 7, the number of the folded portions 25 disposed between the other connection portions 30 adj acent around one connection portion 30 serving as the center is two in a first range A1. In contrast, the number is four in a second range A2. Accordingly, as one of the factors, the reason is that when the stent 10 expands, a greater repulsive force is generated in the first range A1 having the smaller number of the folded portions 25 compared to the second range A2. Then, the tensile direction T of the tensile force applied to the connection portion 30 when the stent 10 according to the present embodiment expands tilts at the angle θ in a direction where the helix of the stent body 20 is tightened. For example, the angle θ is 30 degrees to 80 degrees. In this way, in the stent 10, depending on conditions such as structures and the like, the tensile direction T of the tensile force applied to the connection portion 30 is defined. The tensile direction T can be identified by analysis or experiments.

Then, the first connection portion 31 and the second connection portion 32 which are connected to each other in the stent 10 according to the present embodiment are overlapped and arranged so as to become close to each other when being twisted in a direction where the helix is tightened. Therefore, when the stent 10 expands, even if the tensile force is applied to the connection portion 30 in the direction where the helix is tightened, the force is received in the direction where the first connection portion 31 and the second connection portion 32 become close to each other. Accordingly, both of these are less likely to be disconnected from each other.

In addition, in the first connection portion 31, a first through-hole 37 penetrating toward the central axis of the stent 10 is formed in the first protruding portion 33. In the second connection portion 32, a second through-hole 38 penetrating toward the central axis of the stent 10 is formed in the second protruding portion 35.

The connection member 40 is formed of a biodegradable material, covers the first connection portion 31 and the second connection portion 32, and fills a gap between the first protruding portion 33 and the second housing portion 36, a gap between the second protruding portion 35 and the first housing portion 34, the first through-hole 37, and the second through-hole 38. The connection member 40 is configured to include a biodegradable material which is softer than a material configuring the stent body 20 and the connection portion 30. The stent body 20 having a helical shape is provided with proper rigidity. While the stent 10 is provided with a sufficient vasodilation holding force, and the stent 10 is provided with following property along the deformation of a body lumen such as the blood vessel and the like. In addition, since the connection member 40 covers the first connection portion 31 and the second connection portion 32, and fills the first through-hole 37 and the second through-hole 38, it is possible to prevent the first connection portion 31 and the second connection portion 32 from being disconnected from each other by improving the strength of the connection member 40 joining the first connection portion 31 and the second connection portion 32. In addition, since the connection member 40 is formed of the biodegradable material, the connection member 40 is biodegraded after the stent 10 indwells the body lumen, and is disconnected from the connection portion 30. Accordingly, the stent 10 is allowed to have excellent flexibility, thereby achieving improved following property along the deformation of the body lumen.

In a case of the stent body 20, although dimensions vary depending on an indwelling target portion, the outer diameter when the stent body 20 expands (when the diameter is not decreased) is 1.5 mm to 30 mm, and preferably 2.0 mm to 20 mm. The thickness is 0.04 mm to 1.0 mm, and preferably 0.06 mm to 0.5 mm. The length is 5 mm to 250 mm, and preferably 8 mm to 200 mm. The pitch of the helix (distance between the adjacent helical members 21) is 0.5 mm to 3 mm, and preferably 0.8 mm to 1.5 mm. The axially overlapped length L1 is 0.01 mm to 1 mm, and preferably 0.1 mm to 0.5 mm. The circumferentially overlapped length L2 is 0. 005 mm to 0.2 mm, and preferably 0.01 mm to 0.05 mm. The thickness of the connection member 40, that is, the length of the gap between the first protruding portion 33 and the second housing portion 36 and the length of the gap between the second protruding portion 35 and the first housing portion 34, is 0.005 mm to 0.1 mm, and preferably 0.01 mm to 0.05 mm.

Then, it is preferable to integrally form the stent body 20 in a substantially cylindrical shape, the connection portion 30, and the ring members 26 and 27 by using a non-biodegradable metal material, for example, metal materials of stainless steel, a cobalt-based alloy such as a cobalt-chromium alloy and the like, elastic metal such as a platinum-chromium alloy and the like, or a super elastic alloy such as nickel-titanium alloy and the like.

The stent body 20, the connection portion 30, and the ring members 26 and 27 are manufactured in such a way that non-configuration portions of the stent body 20 and the like are removed by using a metal pipe, thereby configuring an integrally formed product. The stent body 20, the connection portion 30, and the ring members 26 and 27 which use the metal pipe can be formed by means of cutting work (for example, mechanical polishing or laser cutting), electrical discharge machining, chemical etching, or the like. Furthermore, a combination thereof may also be used.

The connection member 40 is formed of a biodegradable material such as a biodegradable polymer material, a biodegradable metal material and the like. For example, as the biodegradable polymer material, it is preferable to use a biodegradable synthetic polymer material such as polylactic acid, polyglycolic acid, lactic-glycolic acid copolymer, polycaprolactone, lactic acid-caprolactone copolymer, glycolic acid-caprolactone copolymer, poly-y-glutamic acid, and the like, or a biodegradable natural polymer material such as cellulose, collagen, and the like. In addition, for example, as the biodegradable metal material, it is preferable to use magnesium, zinc, or the like.

When the connection portion 30 is filled with and covered with the connection member 40, a coating solution prepared by dissolving the connection member 40 in a solvent is applied by using a pivot or the like, for example. The solvent is evaporated, and the connection member 40 can be formed after being dried and solidified.

The solvent is not particularly limited, but it is preferable to use an organic solvent such as methanol, ethanol, dioxane, tetrahydrofuran, dimethylformamide, acetonitrile, dimethyl sulfoxide, acetone, and the like.

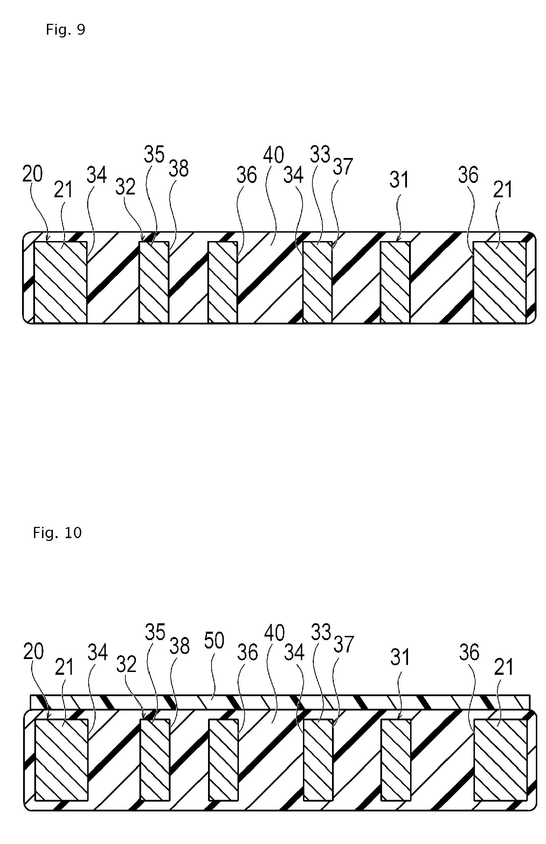

In addition, as a modification example of the stent 10 according to the present embodiment, as illustrated in Fig. 8, without covering the surrounding area of the first connection portion 31 and the second connection portion 32, the connection member 40 may fill a gap between the first protruding portion 33 and the second housing portion 36, a gap between the second protruding portion 35 and the first housing portion 34, the first through-hole 37, and the second through-hole 38. In addition, as illustrated in Fig. 9, the connection member 40 may cover only an outer surface of the first connection portion 31 and the second connection portion 32. In addition, the connection member 40 may cover the entire outer surface of the stent body 20 in addition to the outer surface of the first connection portion 31 and the second connection portion 32.

In addition, as a modification example of the stent 10 according to the present embodiment, as illustrated in Fig. 10, a cover body 50 including a drug may cover an outer surface on a side where the stent body 20, the connection portion 30, and the connection member 40 come into contact with a body lumen.

The cover body 50 includes the drug and a drug loading member for loading the drug. The cover body 50 may be configured to include only the drug without including the drug loading member. The cover body 50 may cover the entire outer surface of the stent 10, and may cover only a portion of the outer surface. In addition, the cover body 50 may also cover both side surfaces interposing the outer surface of the connection member 40 or the like therebetween, or an inner surface opposite to the outer surface.

For example, the drug included in the cover body 50 includes anticancer drugs, immunosuppressive drugs, antibiotics, anti-rheumatic drugs, anti-thrombotic drugs, HMG-CoA reductase inhibitors, insulin resistance improving drugs, ACE inhibitors, calcium antagonists, anti-hyperlipidemic drugs, integrin inhibitors, anti-allergic drugs, anti-oxidants, GP IIb/IIIa antagonists, retinoids, flavonoids, carotenoids, lipid improving drugs, DNA synthesis inhibitors, tyrosine kinase inhibitors, antiplatelet drugs, anti-inflammatory drugs, biologically-derived materials, interferon, and nitric oxide production-promoting substances.

For example, the anticancer drugs include vincristine, vinblastine, vindesine, irinotecan, pirarubicin, paclitaxel, docetaxel, and methotrexate. For example, the immunosuppressive drugs include sirolimus, sirolimus derivatives such as everolimus, pimecrolimus, zotarolimus, biolimus, AP23573, CCI-779, and the like, tacrolimus, azathioprine, cyclosporine, cyclophosphamide, mycophenolate mofetil, gusperimus, mizoribine, and doxorubicin.

For example, the antibiotics include mitomycin, actinomycin, daunorubicin, idarubicin, pirarubicin, aclarubicin, epirubicin, peplomycin, zinostatin stimalamer, and vancomycin. For example, the anti-rheumatic drugs include methotrexate, sodium thiomalate, penicillamine, or lobenzarit. For example, the anti-thrombotic drugs include heparin, aspirin, anti-thrombin, ticlopidine, and hirudin.

For example, the HMG-CoA reductase inhibitors include cerivastatin, cerivastatin sodium, atorvastatin, atorvastatin calcium, rosuvastatin, rosuvastatin calcium, pitavastatin, pitavastatin calcium, fluvastatin, fluvastatin sodium, simvastatin, lovastatin, pravastatin, and pravastatin sodium.

For example, the insulin resistance improving drugs include thiazolidine derivatives such as troglitazone, rosiglitazone, pioglitazone, and the like. For example, the ACE inhibitors include quinapril, perindopril erbumine, trandolapril, cilazapril, temocapril, delapril, enalapril maleate, lisinopril, and captopril. For example, the calcium antagonists include nifedipine, nilvadipine, diltiazem, benidipine, and nisoldipine.

For example, the anti-hyperlipidemic drugs include bezafibrate, fenofibrate, ezetimibe, torcetrapib, pactimibe, K-604, implitapide, and probucol.

For example, the integrin inhibitors include AJM300. For example, the anti-allergic drugs include tranilast. For example, the anti-oxidants include α-tocopherol, catechin, dibutyl hydroxy toluene, and butyl hydroxy anisole. For example, the GP IIb/IIIa antagonists include abciximab. For example, the retinoids include all-trans-retinoic acid. For example, the flavonoids include epigallocatechin, anthocyanins, and proanthocyanidins. For example, the carotenoids include β-carotene and lycopene.

For example, the lipid improving drugs include eicosapentaenoic acid. For example, the DNA synthesis inhibitors include 5-FU. For example, the tyrosine kinase inhibitors include genistein, tyrphostin, erbstatin, and staurosporine. For example, the antiplatelet drugs include ticlopidine, cilostazol, and clopidogrel. For example, the anti-inflammatory drugs include steroids such as dexamethasone, prednisolone, and the like.

For example, the biologically-derived materials include an epidermal growth factor (EGF), a vascular endothelial growth factor (VEGF), a hepatocyte growth factor (HGF), a platelet derived growth factor (PDGF), and a basic fibroblast growth factor (BFGF). For example, the interferon includes interferon-γ1a. For example, the nitric oxide production-promoting substances include L-arginine.

It is preferable to use paclitaxel, docetaxel, sirolimus, and everolimus in view of the fact that all of these are generally used for stenosis treatment in the blood vessel and can be efficiently shifted into cells in a short time. In particular, it is preferable to use sirolimus and paclitaxel.

As the drug loading member, it is preferable to use a polymer material, and particularly preferable to use a biodegradable polymer material which is biodegraded inside a body. After the stent 10 indwells a body lumen, if the biodegradable polymer material loading the drug is biodegraded, the drug is gradually released, thereby preventing restenosis in a stent indwelling area. As the biodegradable polymer material, it is possible to employ a material which is the same as that of the above-described connection member 40.

At covered with the cover body 50, the outer surface of the stent body 20, the connection portion 30, and the connection member 40 are coated with a coating solution prepared by dissolving the drug and the drug loading member in a solvent. The solvent is evaporated, and the drug and the drug loading member can be covered after being dried and solidified.

The solvent is not particularly limited, but it is preferable to use an organic solvent such as methanol, ethanol, dioxane, tetrahydrofuran, dimethylformamide, acetonitrile, dimethyl sulfoxide, acetone, and the like.

The thickness of the cover body 50 is 1 µm to 300 µm, and is preferably 3 µm to 30 µm.

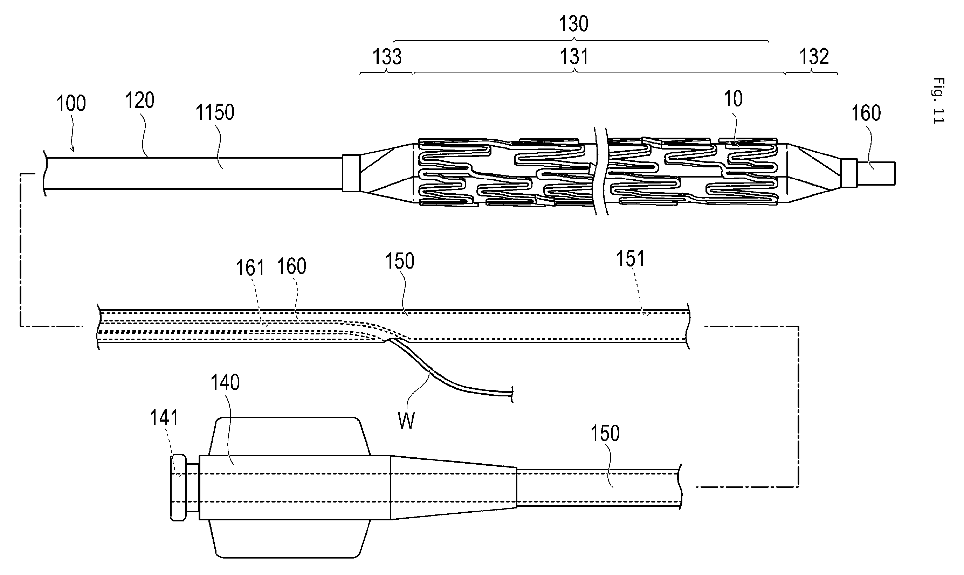

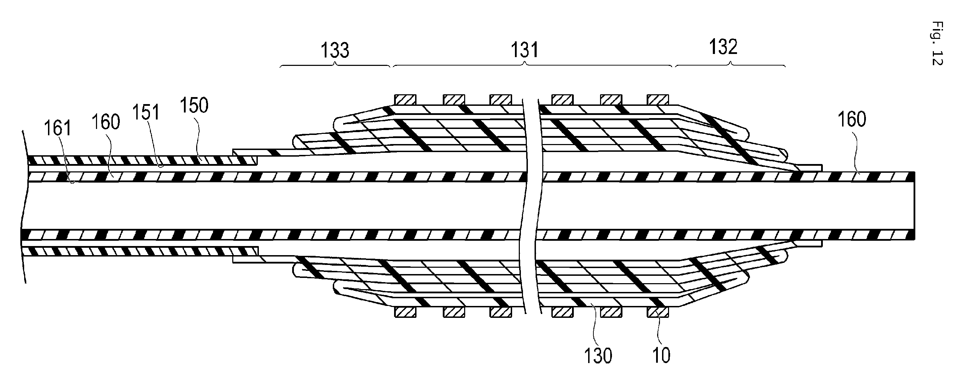

Next, a method of causing the stent 10 according to the present embodiment to indwell a body lumen will be described by exemplifying a case where the stent 10 is caused to indwell the blood vessel. When the stent 10 is caused to indwell, a balloon catheter 100 illustrated in Figs. 11 and 12 is used.

The balloon catheter 100 has an elongated catheter main body 120, a balloon 130 disposed in a distal portion of the catheter main body 120, and a hub 140 fixedly attached to a proximal end of the catheter main body 120.

The catheter main body 120 includes an outer tube 150 which is a tubular body whose distal end and proximal end are open, and an inner tube 160 which is arranged inside the outer tube 150. The outer tube 150 has a dilating lumen 151 in which a dilating fluid is flowed in order to dilate the balloon 130. The inner tube 160 has a guidewire lumen 161 into which a guidewire W is inserted. The dilating fluid may be either gas or liquid, and for example, a gas such as helium gas, CO₂ gas, O₂ gas, and the like, or a liquid such as a physiological salt solution, a contrast medium, and the like may be used.

The distal portion of the inner tube 160 penetrates the inside of the balloon 130, and is open on the distal side beyond the balloon 130. The proximal side thereof penetrates a side wall of the outer tube 150, and is fixedly attached to the outer tube 150 in a liquid-tight manner using an adhesive or by means of heat sealing.

The hub 140 includes a proximal opening portion 141 functioning as a port which communicates with the dilating lumen 151 of the outer tube 150 so as to flow the dilating fluid. The proximal portion of the outer tube 150 is fixedly attached to the hub 140 in a liquid-tight manner using an adhesive, by means of heat sealing, or by using a fastener (not illustrated), for example.

It is preferable to form the outer tube 150 and the inner tube 160 by using a material which is flexible to some degree. For example, as the material, polyolefin such as polyethylene, polypropylene, polybutene, ethylene-propylene copolymer, an ethylene-acetate vinyl copolymer, ionomer, a mixture of two or more of these materials and the like, thermoplastic resin such as soft polyvinyl chloride resin, polyamide, polyamide elastomer, polyester, polyester elastomer, polyurethane, fluorine resin, and the like, silicone rubber, latex rubber, or the like can be used.

As a configuration material of the hub 140, it is possible to preferably use thermoplastic resin such as polycarbonate, polyamide, polysulfone, polyacrylate, methacrylate-butylene-styrene copolymer, or the like.

In order to efficiently spread out a predetermined range when the balloon 130 dilates, the balloon 130 has a cylindrical portion 131 which is formed in a substantially cylindrical shape in the axially central portion and which has substantially the same diameter. A first decreased diameter portion 132 whose diameter decreases in a tapered shape toward the distal side is disposed on the distal side of the cylindrical portion 131 of the balloon 130. A second decreased diameter portion 133 whose diameter decreases in a tapered shape toward the proximal side is disposed on the proximal side thereof.

The distal side of the first decreased diameter portion 132 is fixedly attached to an outer wall surface of the inner tube 160 in a liquid-tight manner using an adhesive or by means of heat sealing. The proximal side of the second decreased diameter portion 133 is fixedly attached to an outer wall surface of the distal portion of the outer tube 150 in a liquid-tight manner using an adhesive or by means of heat sealing. Therefore, the inside of the balloon 130 can communicate with the dilating lumen 151 formed in the outer tube 150, and the dilating fluid can flow into the balloon 130 from the proximal side via the dilating lumen 151. The balloon 130 is caused to dilate by the dilating fluid flowing into the balloon 130. The balloon 130 is brought into a folded state by discharging the dilating fluid flowing into the balloon 130.

In a state where the balloon 130 is not dilated, the balloon 130 is shaped in a state of being folded so as to be wound around the outer peripheral surface of the inner tube 160 in the circumferential direction. The balloon 130 shaped in this way can be molded by means of blow molding in which a tube material is pressed into a mold after being heated and pressurized inside the mold so as to be dilated by the fluid flowing from the inside.

It is preferable to form the balloon 130 by using a material which is flexible to some degree. For example, as the material, polyolefin such as polyethylene, polypropylene, polybutene, ethylene-propylene copolymer, ethylene-acetate vinyl copolymer, ionomer, a mixture of two or more of these materials and the like, thermoplastic resin such as soft polyvinyl chloride resin, polyamide, polyamide elastomer, polyester, polyester elastomer, polyurethane, fluorine resin and the like, silicone rubber, latex rubber, or the like can be used.

When the stent 10 is caused to indwell the blood vessel by using the balloon catheter 100, for example, before the stenosis in the blood vessel is treated, air inside the balloon 130 0 and the dilating lumen 151 is first removed as much as possible. The air inside the balloon 130 and the dilating lumen 151 is replaced with the dilating fluid. In this case, the balloon 130 is in a folded state. In addition, the air inside the inner tube 160 is replaced with a physiological salt solution.

Next, a sheath is caused to indwell the blood vessel of a patient by means of a Seldinger technique, for example. In a state where the guidewire W is inserted into the guidewire lumen 161, the guidewire W and the balloon catheter 100 are inserted into the blood vessel through the inside of the sheath. Subsequently, while the guidewire W is caused to move ahead, the balloon catheter 100 is moved forward so that the balloon 130 reaches the stenosis.

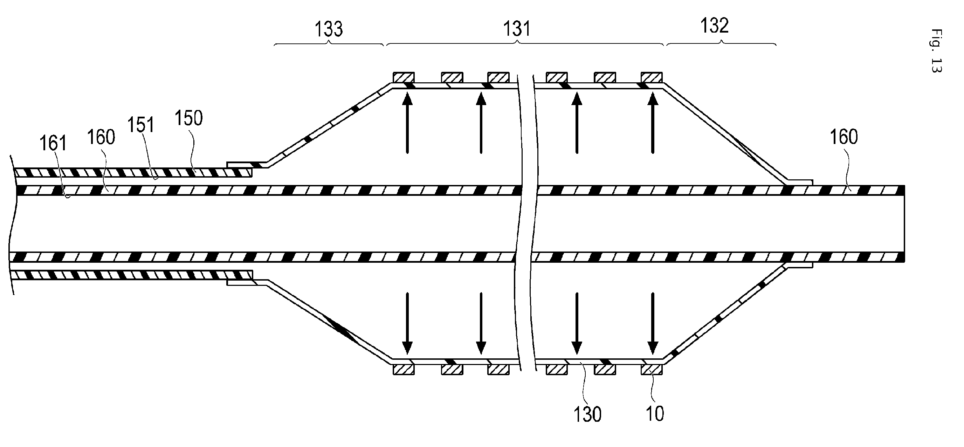

Next, in a state where the balloon 130 is located in the stenosis, a predetermined amount of the dilating fluid is injected through the proximal opening portion 141 of the hub 140 by using an indeflator, a syringe, a pump, or the like. The dilating fluid is fed into the balloon 130 through the dilating lumen 151, thereby dilating the folded balloon 130. In this manner, as illustrated in Fig. 13, the cylindrical portion 131 of the balloon 130 spreads out the stenosis, and plastically deforms and spreads out the stent 10. Accordingly, it is possible to satisfactorily maintain a state where the stenosis is spread out by the stent 10.

Thereafter, the dilating fluid is aspirated and discharged through the proximal opening portion 141, and the balloon 130 is brought into a deflated and folded state. The stent 10 is caused to indwell the stenosis while the stent 10 spreads out the stenosis. Thereafter, the guidewire Wand the balloon catheter 100 are removed from the blood vessel via the sheath. In this manner, manual skills are completed. The entire body of the stent 10 which is caused to indwell the body lumen is covered with endothelial cells with the lapse of time. If the cover body 50 is disposed thereon, the drug included in the cover body 50 is gradually eluted, thereby preventing the occurrence of the restenosis.

Then, the stent 10 according to the present embodiment includes a strengthened expansion holding force since the linear configuration elements configuring the stent 10 are connected to each other by the connection portion 30, and the connection member 40 which connects the first connection portion 31 and the second connection portion 32 to each other includes a biodegradable material. Accordingly, the connection member 40 is biodegraded after the stent 10 indwells the body lumen and a predetermined period of time elapses, and the first connection portion 31 and the second connection portion 32 which are connected by the connection portion 30 are disconnected from each other, thereby achieving excellent flexibility and achieving improved following property along the deformation of the blood vessel and the like. Then, positions in the axial direction X and the circumferential direction Y of the first connection portion 31 and the second connection portion 32 which are connected to each other overlap. Accordingly, for example, when the stent 10 is crimped on the balloon 130, when the stent 10 passes through a lesion after being inserted into the body lumen such as the blood vessel and the like, or when the stent 10 is operated so as to expand, it is possible to prevent the first connection portion 31 and the second connection portion 32 from being unexpectedly disconnected from each other. The first connection portion 31 and the second connection portion 32 are less likely to be unexpectedly disconnected from each other. Accordingly, a vasodilation holding force of the stent 10 is satisfactorily maintained, and the stent 10 can be expanded into a desired shape.

In addition, the connection member 40 is formed by using the filling material for filling a portion between the first connection portion 31 and the second connection portion 32 which are connected to each other. Accordingly, the connection member 40 can be firmly connected to the first connection portion 31 and the second connection portion 32.

In addition, the first connection portion 31 and the second connection portion 32 which are connected to each other have the first protruding portion 33 and the second protruding portion 35 which are formed so as to protrude from the stent body 20, and the concaved second housing portion 36 and the first housing portion 34 which house the first protruding portion 33 and the second protruding portion 35. Accordingly, since the first protruding portion 33 and the second protruding portion 35 are respectively housed in the second housing portion 36 and the first housing portion 34, the first connection portion 31 and the second connection portion 32 are firmly connected to each other, and is less likely to be disconnected from each other.

In addition, the stent body 20 includes the multiple helical members 21 formed in a helical shape extending in the axial direction X of the stent 10, and the first connection portion 31 and the second connection portion 32 connect the adjacent helical members 21 to each other. Accordingly, the stent 10 is provided with flexibility by the helical member 21 having the helical shape, and the stent 10 is provided with proper rigidity by the connection portion 30. In this manner, the stent 10 achieves improved following property along the deformation of the body lumen such as the blood vessel and the like while including a sufficient vasodilation holding force.

In addition, the first connection portion 31 and the second connection portion 32 which are connected to each other are arranged so as to become close to each other when being twisted in the direction where the helix is tightened. Therefore, even when a tensile force is applied to the first connection portion 31 and the second connection portion 32 in the direction where the helix is tightened, the force is received in the direction where the first connection portion 31 and the second connection portion 32 become close to each other. Accordingly, the first connection portion 31 and the second connection portion 32 is less likely to be disconnected from each other.

In addition, the helical members 21 adjacent to each other in the axial direction X are connected to each other only by the first connection portion 31 and the second connection portion 32, except for a portion to be connected in the circumferential direction Y so as to configure one helix. Accordingly, the helical members 21 are not connected to each other so as to be continuously linked in the axial direction X by an integral configuration with the stent body 20 such as the link members 26 and 27, but are connected to each other in the axial direction X only by the first connection portion 31 and the second connection portion 32 between which the filling member 40 is interposed. Therefore, it is possible to improve following property of the stent 10 along the deformation of the body lumen such as the blood vessel and the like by improving flexibility of the stent 10.

In addition, the stent body 20 includes the annular bodies 23 and 24 formed endlessly in both end portions of the helical member 21. Accordingly, a vasodilation holding force can be strongly maintained in both end portions of the stent 10.

In addition, the first connection portion 31 and the second connection portion 32 respectively have the first through-hole 37 and the second through-hole 38 which are to be filled with the connection member 40. Accordingly, the connection member 40 can be more firmly connected to the first connection portion 31 and the second connection portion 32, and the first connection portion 31 and the second connection portion 32 is less likely to be disconnected from each other.

In addition, the stent body 20 is formed of a non-biodegradable metal material. Accordingly, the stent 10 can include more sufficient vasodilation holding force.

Without being limited to the above-described embodiment, the present invention can be modified in various ways within the technical idea of the present invention by those skilled in the art. For example, the biodegradable material configuring the connection member 40 may include a drug. As the drug, the drug applicable to the above-described cover body 50 is applicable. If the drug is included in the connection member 40, the connection member 40 is biodegraded, and the drug is gradually eluted. Accordingly, it is possible to prevent the restenosis of the blood vessel from generating.

In addition, according to the above-described embodiment, the first connection portion 31 and the second connection portion 32 which are connected to each other are arranged so as to become close to each other when being twisted in the direction where the helix is tightened. However, the first connection portion 31 and the second connection portion 32 may be arranged so as to become close to each other when being twisted in the direction where the helix is loosened. According to this form, when a tensile force is applied to the first connection portion and the second connection portion in the direction where the helix is loosened, the force is received in the direction where the first connection portion and the second connection portion become close to each other. Accordingly, the first connection portion and the second connection portion is less likely to be disconnected from each other.

In addition, without being configured to include one helical wire, the stent body may be formed so that two or more wires are arrayed in parallel. In addition, instead of the helical shape, the stent body may be formed so that annular bodies having an endless shape are arrayed and connected to each other by the connection portion in the axial direction X. In this case, it is preferable to dispose a non-biodegradable link member for connecting the annular bodies to each other so that the annular bodies aren't separated from each other after the filling member is biodegraded.

In addition, in the stent 10 according to the above-described embodiment, the first protruding portion 33 is housed in the second housing portion 36, the second protruding portion 35 is housed in the first housing portion 34, and one connection portion 30 includes two sets of protruding portions and housing portions. However, one connection portion may include one set of the protruding portion and the housing portion, or may include three or more sets of protruding portions and housing portions.

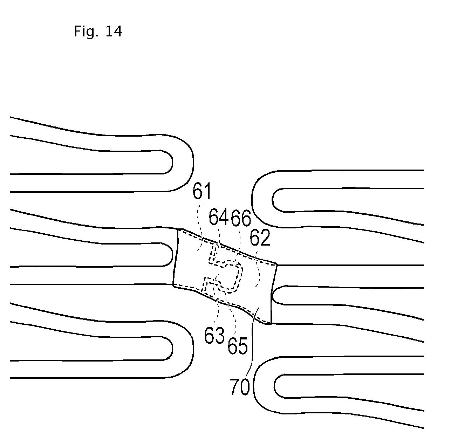

In addition, as in a modification example of the stent illustrated in Fig. 14, a protruding portion 63 may be formed in a first connection portion 61, and a housing portion 64 may be formed in a second connection portion 62. The protruding portion 63 may be housed so that the protruding portion 63 cannot disengage from the housing portion 64 in an in-plane direction (direction parallel to a plane on which the first connection portion 61 and the second connection portion 62 are located). A wide portion 65 whose width is wider than that of a proximal end portion is formed in a distal end portion of the protruding portion 63, and a housing recess 66 for housing the wide portion 65 so that the wide portion 65 cannot disengage from the housing recess 66 is formed in a bottom portion of the housing portion 64. Then, the first connection portion 61 and the second connection portion 62 are connected to each other by a connection member 70 including a biodegradable material (specifically, a filling member formed of the biodegradable material fills a portion between the first connection portion 61 and the second connection portion 62). According to this configuration, the first connection portion 61 and the second connection portion 62 are firmly connected to each other, and can be much less likely to be disconnected from each other.

In addition, a through-hole filled with the connection member may not be disposed in the first connection portion and the second connection portion.

In addition, the stent may be a self-expandable stent which expands by using its own elastic force.

### [Reference Signs List]

- 10: STENT,
- 20: STENT BODY,
- 21: HELICAL MEMBER,
- 25: FOLDED PORTION,
- 30: CONNECTION PORTION,
- 31, 61: FIRST CONNECTION PORTION,
- 32, 62: SECOND CONNECTION PORTION,
- 33: FIRST PROTRUDING PORTION (PROTRUDING PORTION),
- 34: FIRST HOUSING PORTION (HOUSING PORTION),
- 35: SECOND PROTRUDING PORTION (PROTRUDING PORTION),
- 36: SECOND HOUSING PORTION (HOUSING PORTION),
- 37: FIRST THROUGH-HOLE (THROUGH-HOLE)
- 38: SECOND THROUGH-HOLE (THROUGH-HOLE)
- 40, 70: CONNECTION MEMBER,
- 63: PROTRUDING PORTION,
- 64: HOUSING PORTION,
- L1: AXIALLY OVERLAPPED LENGTH,
- L2: CIRCUMFERENTIALLY OVERLAPPED LENGTH
- T: TENSILE DIRECTION,
- X: AXIAL DIRECTION,
- Y: CIRCUMFERENTIAL DIRECTION

## Claims

1. A stent comprising:
a stent body that is configured to include linear configuration elements, and that has a cylindrical shape having a gap as a whole;
a first connection portion and a second connection portion which are integrally formed with the stent body in each of the linear configuration elements adjacent to each other side by side in an axial direction of the stent body having the cylindrical shape, and which are connected to each other while positions overlap in the axial direction and in a circumferential direction of the stent body; and
a connection member that includes a biodegradable material for connecting the first connection portion and the second connection portion to each other.

2. The stent according to Claim 1,
wherein the connection member is a filling member for filling a portion between the first connection portion and the second connection portion which are connected to each other.

3. The stent according to Claim 1 or 2,
wherein at least one of the first connection portion and the second connection portion which are connected to each other has a protruding portion formed so as to protrude from the stent body, and at least the other one of the first connection portion and the second connection portion has a recessed housing portion for housing the protruding portion.

4. The stent according to any one of Claims 1 to 3,
wherein the stent body includes multiple helical members formed in a helical shape wound around the stent body in the axial direction, and the first connection portion and the second connection portion connect the adj acent helical members to each other.

5. The stent according to Claim 4,
wherein the first connection portion and the second connection portion which are connected to each other are arranged so as to become close to each other when being twisted in a direction where the helix is tightened.

6. The stent according to Claim 4,
wherein the first connection portion and the second connection portion which are connected to each other are arranged so as to become close to each other when being twisted in a direction where the helix is loosened.

7. The stent according to any one of Claims 4 to 6,
wherein the helical members adjacent to each other in the axial direction are connected to each other only by the first connection portion and the second connection portion, except for a portion connected in the circumferential direction so as to configure the same helix.

8. The stent according to any one of Claims 4 to 7,
wherein the stent body further includes an annular body formed endlessly in both ends of the helical member.

9. The stent according to any one of Claims 1 to 8,
wherein the first connection portion and the second connection portion have a through-hole filled with the connection member.

10. The stent according to any one of Claims 1 to 9,
wherein the connection member includes a drug.

11. The stent according to any one of Claims 1 to 10,
wherein the stent body is formed of a non-biodegradable metal material.
